# EUROPEAN PATENT SPECIFICATION

(11) **EP 1 543 134 B1**
(45) Date of publication and mention of the grant of the patent: **18.04.2007**
(21) Application number: 03757854.9
(22) Date of filing: 16.09.2003
(51) Int. Cl.: C12P 7/26, C12R 1/865, C12R 1/74, C12R 1/645, C12R 1/15

(54) **PROCESS FOR ACTINOL PRODUCTION FROM KETOISOPHORONE**
VERFAHREN ZUR HERSTELLUNG VON AKTINOL AUS KETOISOPHORON
PROCEDE DE PRODUCTION D'ACTINOL A PARTIR DE CETOISOPHORONE

(30) Priority: 27.09.2002 EP 02021605
(43) Date of publication of application: 22.06.2005
(73) Proprietor: DSM IP Assets B.V., 6411 TE Heerlen (NL)
(72) Inventor: Hoshino, Tatsuo, Kanagawa, Kanagawa 248-0027 (JP); Setoguchi, Yutaka, Kanagawa, Kanagawa 251-0033 (JP); Shimizu, Sakayu, Kyoto-shi, Kyoto 616-8212 (JP); Tabata, Kazuyuki, Kanagawa-ken, Kanagawa 253-0002 (JP)
(74) Representative: Keller, Günter
(86) International application number: PCT/EP2003/010295
(87) International publication number: WO 2004/029263

(56) References cited:
- EP-A- 0 982 406
- EP-A- 1 026 235
- EP-A- 1 074 630
- EP-A- 1 122 315
- US-A- 4 072 715
- WADA M ET AL: "Purification and characterization of monovalent cation-activated levodione reductase from Corynebacterium aquaticum M-13." APPLIED AND ENVIRONMENTAL MICROBIOLOGY, vol. 65, no. 10, October 1999 (1999-10), pages 4399-4403, XP002272891 ISSN: 0099-2240
- WANNER P ET AL: "Purification and characterization of two enone reductases from Saccharomyces cerevisiae" EUROPEAN JOURNAL OF BIOCHEMISTRY, vol. 255, no. 1, July 1998 (1998-07), pages 271-278, XP002202649 ISSN: 0014-2956
- YOSHISUMI A ET AL: "Cloning, sequence analysis, and expression in Escherichia coli of the gene encoding monovalent cation-activated levodione reductase from Corynebacterium aquaticum M-13." BIOSCIENCE, BIOTECHNOLOGY, AND BIOCHEMISTRY, vol. 65, no. 4, April 2001 (2001-04), pages 830-836, XP001160832 ISSN: 0916-8451
- STOTT K ET AL: "Old Yellow Enzyme: The discovery of multiple isozymes and a family of related proteins" JOURNAL OF BIOLOGICAL CHEMISTRY, vol. 268, no. 9, 25 March 1993 (1993-03-25), pages 6097-6106, XP002252405 ISSN: 0021-9258
- NIINO Y S ET AL: "A new Old Yellow Enzyme of Saccharomyces cerevisiae" JOURNAL OF BIOLOGICAL CHEMISTRY, vol. 270, no. 5, 3 February 1995 (1995-02-03), pages 1983-1991, XP002252406 ISSN: 0021-9258
- VAZ A D N ET AL: "Old Yellow Enzyme: Aromatization of cyclic enones and the mechanism of a novel dismutation reaction" BIOCHEMISTRY, vol. 34, no. 13, 1995, pages 4246-4256, XP001164161 ISSN: 0006-2960
- WADA M ET AL: "Production of a doubly chiral compound, (4R,6R)-4-hydroxy-2,2,6-trime thylcyclohexanone, by two-step enzymatic asymmetric reduction." APPLIED AND ENVIRONMENTAL MICROBIOLOGY, vol. 69, no. 2, February 2003 (2003-02), pages 933-937, XP009011700 ISSN: 0099-2240
- KATAOKA M ET AL: "Old Yellow Enzyme from Candida macedoniensis catalyzes the stereospecific reduction of the C=C bond of ketoisophorone." BIOSCIENCE, BIOTECHNOLOGY, AND BIOCHEMISTRY, vol. 66, no. 12, December 2002 (2002-12), pages 2651-2657, XP009011699 ISSN: 0916-8451

## Description

The present invention relates to a one-step process for the microbial production of (4R, 6R)-4-hydroxy-2,2,6-trimethylcyclohexanone (hereinafter referred to as actinol) from 2,6,6-trimethyl-2-cyclohexene-1,4-dione (hereinafter referred to as ketoisophorone) and to recombinant organisms involved in such process.

Actinol is useful for the synthesis of carotenoids, such as zeaxanthin. EP 982,406 discloses an efficient microbial production of actinol which comprises contacting levodione with a microorganism which is selected from the group consisting of microorganisms of the genera *Cellulomonas, Corynebacterium, Planococcus,* and *Arthrobacter,* and which is capable of selective asymmetric reduction of levodione to actinol, and recovering the resulting actinol from the reaction mixture. EP 1,026,235 discloses an enzyme, levodione reductase, that acts on levodione to produce actinol, which was isolated from *Corynebacterium aquaticum* AKU611 (FERM BP-6448). The genetic material such as an isolated DNA comprising a nucleotide sequence coding for an enzyme having levodione reductase activity, a polypeptide encoded by such a DNA, recombinant organisms and the like, are disclosed by EP 1,122,315.

EP 1,074,630 discloses an efficient microbial production of levodione which comprises contacting ketoisophorone with at least one kind of yeast capable of converting ketoisophorone into levodione and selected from the group of species consisting of *Saccharomyces rouxii (Zygosaccharomyces rouxii), Saccharomyces delbrueckii (Saccharomyces unisporus, Torulaspora delbrueckii), Saccharomyces willianus, Zygosaccharomyces bailii,* and *Candida tropicalis.* Isolation and characterization of an enzyme ketoisophorone reductase that acts on ketoisophorone to produce levodione was recently reported.

Wada et al. (1999) Applied and Environmental Microbiology 65 (10), 4399 - 4403 describes the purification and characterization of monovalent cation-activated levodione reductase from *Corynebacterium aquaticum* M-13.

Wanner and Tressl (1998) Eur. J. Biochem. 255, 271 - 278 describes the purification and characterization of two enone reductases from *Saccharomyces cerevisiae.*

Until now there was no biological one-step process for the direct production of optically active actinol from ketoisophorone, which requires two sequential reactions from ketoisophorone to levodione, and levodione to actinol.

The present invention is directed to a process for the production of actinol from ketoisophorone with high optical purity by a single step reaction using a recombinant microorganism which is capable of reducing ketoisophorone to levodione, and levodione to actinol simultaneously.

More particularly, the present invention provides a process for producing actinol from ketoisophorone by contacting ketoisophorone with a recombinant microorganism or cell-free extract thereof which has both ketoisophorone-reducing activity and levodione-reducing activity because of introduction of levodione reductase gene, and isolating the resulted actinol from the reaction mixture.

Further described herein is a process for producing actinol from ketoisophorone by contacting ketoisophorone with both ketoisophorone reductase, which is capable of catalyzing the conversion of ketoisophorone to levodione, and levodione reductase, which is capable of catalyzing the conversion of levodione to actinol, simultaneously.

The present invention further provides a recombinant microorganism, that is obtained by transforming a host microorganism which is capable of reducing ketoisophorone to levodione with levodione reductase gene.

Further described herein is a recombinant microorganism, that is obtained by transforming a host microorganism which is capable of reducing levodione to actinol with ketoisophorone reductase gene.

Further described herein is a recombinant microorganism which expresses both ketoisophorone reductase gene and levodione reductase gene.

In the present invention, any recombinant microorganism which is capable of producing actinol from ketoisophorone can be used. In the present invention, any microorganism which is capable of producing levodione from ketoisophorone can be used as a host microorganism to obtain a recombinant microorganism which is capable of producing actinol from ketoisophorone by introducing levodione reductase gene in it. Said microorganism can also be used as a donor strain for ketoisophorone reductase gene. The microorganism is selected from the group consisting of microorganisms of the genera *Saccharomyces, Zygosaccharomyces,* and *Candida.* Examples include *S*. *cerevisiae* ATCC7754, *S. rouxii (Z. rouxii)* HUT7191 (IFO 0494), *S. delbrueckii* HUT7116 (*S*. *unisporus* IFO 0298), *S. delbrueckii (Torulaspora delbrueckii)* HUT7102, *S. willianus* HUT7106, *Z*. *bailii* ATCC11486, *C. tropicalis* IFO 1403, mutants thereof, and even commercially available baker's yeast (available from e.g. the Oriental Yeast Co., Ltd.). These yeasts are disclosed in EP 1,074,630, and have been deposited with at least one of the depositaries American Type Culture Collection (ATCC), 10801 University Boulevard Manassae, VA20100-2209, USA; HUT Culture Collection Room, Department of Fermentation Technology, Hiroshima University; and Institute for Fermentation Osaka (IFO), 17-85 Jusohonmachi 2-chome, Yodogawa-ku, Osaka, Japan. Each is available from the pertinent depositary to anyone upon request.

Any microorganism which is capable of producing actinol from levodione can be used as a host microorganism to obtain a recombinant microorganism which is capable of producing actinol from ketoisophorone by introducing ketoisophorone reductase gene in it. Said microorganism can also be used as a donor strain for levodione reductase gene. A preferred microorganism is selected from the group consisting of microorganisms of the genera *Cellulomonas, Corynebacterium, Planococcus,* and *Arthrobacter,* including *Cellulomonas sp.* AKU672 (FERM BP-6449), *Corynebacterium aquaticum* AKU610 (FERM BP-6447), *C. aquaticum* AKU611 (FERM BP-6448), *P*. *okeanokoites* AKU152 (IFO 15880), *A. sulfureus* AKU635 (IFO 12678), and mutants thereof. These microorganisms are disclosed in EP 982,406. *Cellulomonas sp.* AKU672 (FERM BP-6449), *Corynebacterium aquaticum* AKU610 (FERM BP-6447) and *C*. *aquaticum* AKU611 (FERM BP-6448) were deposited at the National Institute of Advanced Industrial Science and Technology (AIST), Tsukuba Central 6, 1-1-1 Higashi, Tsukuba, Ibaraki 305-8566, Japan, in the name of F. Hoffmann-La Roche AG of Grenzacherstrasse 124, CH-4070 Basle, Switzerland on August 4, 1998, under Budapest Treaty. *P*. *okeanokoites* AKU152 (IFO 15880) and *A. sulfureus* AKU635 (IFO 12678) were deposited at IFO under the deposit No. described above, and any one can publicly obtain them from the depository. One of the most preferred strains is *C*. *aquaticum* AKU611.

Any microorganism which is originally not capable of producing levodione from ketoisophorone, nor actinol from levodione, can be used as a host microorganism to obtain a recombinant microorganism which is capable of producing actinol from ketoisophorone by introducing both genes encoding ketoisophorone reductase and levodione reductase in it.

Either a growing or a resting cell culture or immobilized cells or a cell-free extract, or the like, of said recombinant microorganism may be used for the production of actinol. A growing cell culture can be obtained by culturing a recombinant microorganism in a nutrient medium containing saccharides such as glucose or sucrose, alcohols, such as ethanol or glycerol, fatty acids, such as oleic acid and stearic acid or esters thereof, or oils, such as rapeseed oil or soybean oil, as carbon sources; ammonium sulfate, sodium nitrate, peptone, amino acids, corn steep liquor, bran, yeast extract and the like, as nitrogen sources; magnesium sulfate, sodium chloride, calcium carbonate, potassium monohydrogen phosphate, potassium dihydrogen phosphate, and the like, as inorganic salt sources; and malt extract, meat extract, and the like, as other nutrient sources.

Cultivation of the recombinant microorganism can be carried out aerobically or anaerobically at pH values from 4.0 to 9.0, at a temperature in the range of from 10 to 50°C for 15 min to 72 hours, preferably, at pH values from 5.0 to 8.0, at a temperature in the range of from 20 to 40°C for 30 min to 48 hours.

Using the growing cell culture thus obtained, a resting cell culture or immobilized cell or cell-free extract may be prepared by any means generally known in the art.

The concentration of ketoisophorone in a reaction mixture can vary depending on other reaction conditions, but, in general, may be between 0.1 g/l and 300 g/l, preferably between 1 g/l and 30 g/l.

Actinol can also be produced by contacting ketoisophorone with ketoisophorone reductase and levodione reductase concomitantly.

One of the most preferred levodione reductases and a method for its preparation is described in EP 1,026,235. This enzyme was characterized by the following physicochemical properties:
1) The levodione reductase catalyzes regio- and stereoselective reduction of levodione to actinol.
2) The relative molecular mass of the enzyme is estimated to be 142,000 -155,000 ± 10,000 Da, consisting of four homologous subunits having a molecular mass of 36,000 ± 5,000 Da.
3) The optimum temperature is 15-20°C at pH 7.0 and the optimum pH is 7.5.
4) The enzyme requires NAD⁺ or NADH as a cofactor and is highly activated by monovalent cations, such as K⁺, Na⁺, Cs⁺, Rb⁺, and NH₄⁺.

Levodione reductase catalyzes the reduction of levodione to actinol in the presence of a cofactor according to the following formula:

levodione + NADH ⇄ actinol + NAD

Ketoisophorone reductase and its genetic material such as an isolated DNA comprising a nucleotide sequence coding for an enzyme having ketoisophorone reductase activity can be obtained from a microorganism which is capable of reducing ketoisophorone to levodione. A preferred microorganism is selected from the group consisting of microorganisms of the genera *Saccharomyces, Zygosaccharomyces* and *Candida,* including *S*. *cerevisiae* ATCC7754, *S*. *rouxii (Z. rouxii)* HUT7191 (IFO 0494), *S. delbrueckii* HUT7116 *(S. unisporus* IFO 0298), *S. delbrueckii (Torulaspora delbrueckii)* HUT7102, *S*. *willianus* HUT7106, *Z*. *bailii* ATCC11486, *C*. *tropicalis* IFO 1403, mutants thereof, and even commercially available baker's yeast (available from e.g. the Oriental Yeast Co., Ltd.). These microorganisms are disclosed in EP 1,074,630.

The enzyme reaction may, e.g., be performed as follows: The basal reaction mixture (total volume: 1 ml): 200 µl of 1 M potassium phosphate buffer (pH 7.0), 40 µl of 8 mM NADH in 0.2 mM KOH, 200 µl of ketoisophorone solution, and 20-80 µl of the enzyme solution, and water up to 1 ml, is incubated at pH values of from 4.0 to 9.0, at a temperature range of from 10 to 50°C for 5 min to 48 hours, preferably at pH values of from 5.0 to 8.0, at a temperature range of from 20 to 40°C for 15 min to 24 hours.

The concentration of ketoisophorone in a reaction mixture can vary depending on other reaction conditions, but, in general, may be between 0.1 g/l and 300 g/l, preferably between 1 g/l and 30 g/l.

Actinol produced biologically or enzymatically in the reaction mixture as described above may be extracted by an organic solvent such as ethyl acetate, n-hexane, toluene, or n-butyl to recover the actinol into the organic solvent layer. The extract is analyzed by known methods such as gas chromatography, high performance liquid chromatography, thin layer chromatography or paper chromatography, or the like.

After the reaction, actinol in the reaction mixture may be recovered, for example, by extraction with a water-immiscible organic solvent which readily solubilizes actinol, such as ethyl acetate, n-hexane, toluene or n-butyl acetate. Further purification of actinol can be effected by concentrating the extract to directly crystallize actinol or by the combination of various kinds of chromatography, for example, thin layer chromatography, adsorption chromatography, ion-exchange chromatography, gel filtration chromatography or high performance liquid chromatography.

The following Examples further illustrate the present invention, but these are not thereby limiting the scope of the invention.

### Example 1: Production of levodione by reducing ketoisophorone using S. cerevisiae INVScI resting cells

*S. cerevisiae* INVScI was inoculated into YP-raffinose medium (5 ml in a test tube) containing 10 g/l of Bacto Yeast Extract, 20 g/l of Bacto Peptone, and 20 g/l of raffinose, and cultivated at 30°C for 20 hr. A portion of the seed culture was inoculated into the same medium (10 ml in a test tube) and cultivated at 30°C for 5 hr. Initial optical density value for 600 nm wavelength of the cultivation was adjusted to 0.2. After addition of 20 % galactose solution (1 ml), cultivation was continued for 16 hr. The main culture broth of each test tube was collected and harvested by centrifugation. The pellet of the centrifugation was used as a resting cell of the following ketoisophorone-reducing reaction after wash with 0.1 M of KPB (pH 6.0).
The ketoisophorone-reducing reaction was done as follows. 0.11 g of resting cells were suspended into 1 ml of reaction buffer, which is 0.1 M of KPB (pH 6.0) containing 50 g/l of glucose and 5.0 g/l of ketoisophorone, and incubated at 30°C in a test tube with gently shaking (200 rpm). The reaction solution was recovered by centrifuging the reaction mixture and removing resting cell pellet. Resulting reaction solution was then extracted with equal volume of ethyl acetate and analyzed by gas chromatography [column: BGB-176 (BGB Analytik AG, Switzerland), instrument: GC-14A (SHIMADZU, Japan), carrier gas: He, column temperature: 1°C/min elevation from 100°C to 150°C, injector temperature: 200°C].
The result of the reaction is described in Table 1. After 17.0 hr reaction, 2.8 g/l of levodione was produced from 5.0 g/l of ketoisophorone. Concentration of a major byproduct, (S,R)-isomer of actinol, was analyzed to be 0.7 g/l.

**Table 1: Ketoisophorone-reducing reaction using S. cerevisiae INVScI cells**

| time (hr) | titer (g/l) | | | |
|---|---|---|---|---|
| | ketoisophorone | (R)-levodione | (R,R)-actinol | (S,R)-actinol |
| 0 | 3.60 | 0.00 | 0.00 | 0.00 |
| 1 | 3.69 | 0.00 | 0.00 | 0.02 |
| 2 | 2.65 | 0.53 | 0.00 | 0.05 |
| 4 | 2.25 | 1.35 | 0.00 | 0.12 |
| 6 | 1.06 | 1.94 | 0.01 | 0.18 |
| 17 | 0.00 | 2.78 | 0.07 | 0.65 |
| 19 | 0.00 | 2.58 | 0.07 | 0.60 |

### Example 2: Introduction of the levodione reductase gene derived from C. aquaticum AKU611 into S. cerevisiae INVScI

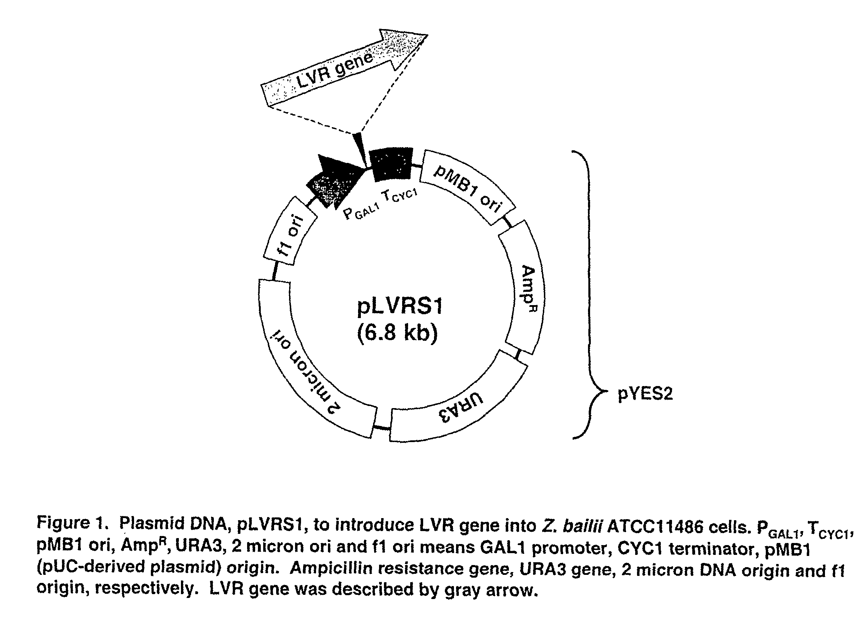

A DNA fragment encoding levodione reductase gene has already been cloned by using PCR primers designed from the information based on partial amino acid sequences of purified levodione reductase as described below.
At first, genomic DNA of *Corynebacterium aquaticum* AKU611 (FERM BP-6448) was prepared using Genome Isolation Kit (BIO101). Using the prepared genomic DNA as template, a complete coding sequence for the levodione reductase gene without excessive flanking region was obtained by PCR amplification using a thermal cycler (Perkin elmer 2400, U.S.A.). The two synthetic primers used were: LV-ORF(+)(SEQ ID NO:1) (having an EcoRI site GAATTC) and LV-ORF(-) (SEQ ID NO:2) (having an PstI site CTGCAG). The PCR mixture (0.02 ml) contained 5 pmol of each primer, 0.2 mM of each dNTP, and 1 U of LA Taq (Takara Shuzo co.LTD / Kyoto, Japan). The initial template denaturation step consisted of 1 min at 94°C. An amplification cycle of 20 sec at 98°C, 2 min at 70°C and 4 min at 72°C was repeated for 25 times.
By this reaction, a DNA fragment containing a complete ORF of the levodione reductase gene (0.8 Kb) was amplified. This amplified levodione reductase gene was treated with EcoRI and PstI, and ligated with a vector, pKK223-3 (Amersham Bioscience / Bucking-hamshire, England) that was predigested with EcoRI and PstI to construct a plasmid, pKKLR(1-15). *E.coli* JM109 was transformed with the ligation mixture, and several clones were selected for sequence analysis. The sequence of the cloned levodione reductase gene of each candidate clone was examined. One of the clones that showed completely the same sequence as the levodione reductase sequence of *Corynebacterium aquaticum* AKU611 (FERM BP-6448) was named as JM109[pKKLR(1-15)]. pKKLR(1-15) was isolated from this strain and used for further experiments.
The 0.8 kb DNA fragment encoding levodione reductase gene was cut out from plasmid pKKLR(1-15) which is comprising levodione reductase gene and *E*. *coli* vector pKK223-3, and inserted into pYES2, which is an expression vector for *S*. *cerevisiae.* GAL1 promoter and CYC1 terminator, both of which are originally existing on pYES2, are connected to the levodione reductase gene on the plasmid as a result of ligation of the 0.8 kb levodione reductase fragment with *Eco*RI-digested form of the plasmid pYES2, followed by blunting of the ligated DNA and self-ligation of the DNA. After confirming the direction of the inserted levodione reductase gene, this plasmid, named as pLVRS1, was used to transform *S. cerevisiae* INVScI. The plasmid is illustrated in the Figure, depicting plasmid DNA, pLVRS1, to introduce LVR gene into *Z*. *bailii* ATCC11486 cells. P_{GAL1}, T_{CYC1}, pMB1 ori, Amp^{R}, URA3, 2 micron ori and f1 ori means GAL1 promoter, CYC1 terminator, pMB1 (pUC-derived plasmid) origin, ampicillin resistance gene, URA3 gene, 2 micron DNA origin and f1 origin, respectively. LVR gene is described by gray arrow.
The levodione reductase-encoding plasmid pLVRSl was introduced into *S*. *cerevisiae* INVScI cells by using S. c. EasyComp Transformation Kit (Invitrogen). The method of the transformation was basically the same as the protocol of the transformation kit. Transformed cells can be selected by spreading resulting transformation solution onto solid synthetic medium which lacks uracil because of the presence of the uracil auxotroph of the parent strain and URA3 marker of the plasmid, and incubating these plates for 4 days at 30°C. Presence of the plasmid in the yeast strain was confirmed by checking occurrence of pLVRS1-containing *E. coli* DH5 alpha strains after transformation of the *E. coli* strain by using total DNA of the candidate of *S. cerevisiae* INVScI transformant strain as a donor DNA. The strain possessing pLVRS1, *S. cerevisiae* (pLVRS1), was then used for further experiments.

### Example 3: Production of actinol by reducing ketoisophorone using S. cerevisiae INVScI resting cells containing the levodione reductase gene derived from C. aquaticum AKU611

The ketoisophorone-reducing reaction using resting cells of *S. cerevisiae* INVScI possessing pLVRSl was originally performed by basically the same method as described in Example 1. The result of the reaction is described in Table 2. After 19.0 hr reaction, 1.6 g/l of actinol was produced from 5.0 g/l of ketoisophorone. Optical purity for actinol was not so high (67.2 %) because of the relatively high value of (S,R)-isomer concentration (0.48 g/l).

**Table 2: Ketoisophorone-reducing reaction using S. cerevisiae INVScI possessing pLVRSl**

| Time (hr) | titer (g/l) | | | | | optical purity for (R,R)-ACT (% e.e) |
|---|---|---|---|---|---|---|
| | KIP | (R)-LDN | (R,R)-ACT | (S,R)-ACT | (S)-PHO | |
| 0 | 3.79 | 0.00 | 0.00 | 0.00 | 0.00 | |
| 1 | 3.02 | 0.00 | 0.03 | 0.01 | 0.33 | 66.6 |
| 2 | 2.44 | 0.39 | 0.12 | 0.03 | 0.52 | 73.0 |
| 4 | 1.61 | 0.83 | 0.31 | 0.07 | 0.62 | 77.8 |
| 6 | 1.00 | 1.12 | 0.55 | 0.12 | 0.60 | 78.8 |
| 17 | 0.11 | 1.24 | 1.31 | 0.36 | 0.29 | 71.0 |
| 19 | 0.06 | 1.72 | 1.60 | 0.48 | 0.27 | 67.2 |

| | | | | | | |
|---|---|---|---|---|---|---|
| KIP: ketoisophorone; (R)-LDN: (R)-levodione; (R,R)-ACT: (R,R)-actinol; (S,R)-ACT: (S,R)-actinol, (S)-PHO: (S)-phorenol | | | | | | |

0.33 g of resting cells were suspended into 1 ml of reaction buffer, which was 0.1 M of KPB (pH 6.0) containing 150 g/l of glucose and 10.0 g/l of ketoisophorone, and incubated at 20°C in a test tube with gently shaking (200 rpm). The reaction solution was recovered, extracted, and analyzed by gas chromatography as described in Example 1.

The result of the reaction is described in Table 3. After 25.0 hr reaction, 3.3 g/l of actinol was produced from 10.0 g/l of ketoisophorone. Since the concentration of (S,R)-isomer could be suppressed to 0.52 g/l, optical purity for actinol was increased to 81.5 %.

**Table 3: Ketoisophorone-reducing reaction using S. cerevisiae INVScI possessing pLVRS1**

| Time (hr) | titer (g/l) | | | | | optical purity for (R,R)-ACT (%e.e) |
|---|---|---|---|---|---|---|
| | KIP | (R)-LDN | (R,R)-ACT | (S,R)-ACT | (S)-PHO | |
| 0 | 11.23 | 0.00 | 0.00 | 0.00 | 0.00 | |
| 1.5 | 6.78 | 0.00 | 0.09 | 0.00 | 0.99 | 100.0 |
| 3.0 | 5.93 | 0.57 | 0.35 | 0.06 | 1.88 | 81.6 |
| 4.5 | 3.86 | 0.86 | 0.65 | 0.09 | 2.10 | 85.9 |
| 6.0 | 2.80 | 0.99 | 0.94 | 0.12 | 2.18 | 85.2 |
| 7.5 | 2.27 | 1.20 | 1.14 | 0.14 | 2.08 | 85.3 |
| 9.0 | 1.77 | 1.23 | 1.34 | 0.16 | 2.06 | 85.4 |
| 22.0 | 0.35 | 1.63 | 3.20 | 0.47 | 1.27 | 82.6 |
| 23.5 | 0.31 | 1.64 | 3.29 | 0.50 | 1.18 | 81.9 |
| 25.0 | 0.25 | 1.51 | 3.34 | 0.52 | 1.08 | 81.5 |
| 26.5 | 0.21 | 1.42 | 3.33 | 0.56 | 0.99 | 80.3 |

| | | | | | | |
|---|---|---|---|---|---|---|
| KIP: ketoisophorone; (R)-LDN: (R)-levodione; (R,R)-ACT: (R,R)-actinol; (S,R)-ACT: (S,R)-actinol, (S)-PHO: (S)-phorenol | | | | | | |

## Claims

1. A process for producing actinol from ketoisophorone which comprises contacting ketoisophorone with a recombinant microorganism or cell-free extract thereof in a reaction mixture,
- wherein said recombinant microorganism is obtainable by transforming a host microorganism which is capable of reducing ketoisophorone to levodione, selected from the group consisting of microorganisms of the genera *Saccharomyces, Zygosaccharomyces,* and *Candida,* such as commercially available baker's yeast, *Saccharomyces cerevisiae* ATCC7754, *Saccharomyces rouxii* HUT7191, *Zygosaccharomyces rouxii* IFO0494, *Saccharomyces delbrueckii* HUT7116, *Saccharomyces unisporus* IFO0298, *Saccharomyces delbrueckii (Torulaspora delbrueckii)* HUT7102, *Saccharomyces willianus* HUT7106, *Zygosaccharomyces bailii* ATCC11486, *Candida tropicalis* IFO1403, and a mutant thereof which is capable of reducing ketoisophorone to levodione, with a levodione reductase gene, derived from a microorganism belonging to any one of the genera *Cellulomonas, Corynebacterium, Planococcus,* and *Arthrobacter,* such as *Cellulomonas* sp. AKU672 FERM BP-6449, *Corynebacterium aquaticum* AKU610 FERM BP-6447, *C.aquaticum* AKU611 FERM BP-6448, *P.okeanokoites* AKU152 IFO15880, *A.sulfureus* AKU635 IFO12678, or a mutant thereof,
- wherein the reaction is carried out at pH values of from 4.0 to 9.0 and at a temperature range from 10 to 50°C and for 15 minutes to 72 hours,
and isolating the produced actinol from the reaction mixture.

2. The process according to claim 1, wherein the reaction is carried out at pH values of from 5.0 to 8.0, and at a temperature range from 20 to 40°C, and for 30 minutes to 48 hours.

3. A recombinant microorganism that is obtainable by transforming a host microorganism which is capable of reducing ketoisophorone to levodione, belonging to the genera *Saccharomyces, Zygosaccharomyces* or *Candida,* such as commercially available baker's yeast, *S.cerevisiae* ATCC7754, *S.rouxii* HUT7191, *Z.rouxii* IFO0494, *S.delbrueckii* HUT7116, *S.unisporus* IFO0298, *S.delbrueckii (Torulaspora delbrueckii)* HUT7102, *S.willianus* HUT7106, *Z.bailii* ATCC11486, *C.tropicalis* IFO1403, and a mutant thereof which is capable of reducing ketoisophorone to levodione, with a levodione reductase gene, derived from a microorganism belonging to any one of the genera *Cellulomonas, Corynebacterium, Planococcus,* and *Arthrobacter,* such as *Cellulomonas* sp. AKU672 FERM BP-6449, *Corynebacterium aquaticum* AKU610 FERM BP-6447, *C.aquaticum* AKU611 FERM BP-6448, *P.okeanokoites* AKU152 IFO15880, *A.sulfureus* AKU635 IFO12678, or a mutant thereof.

## Patentansprüche

1. Verfahren zur Herstellung von Actinol aus Ketoisophoron, das umfasst, dass Ketoisophoron mit einem rekombinanten Mikroorganismus oder einem zellfreien Extrakt davon in einer Reaktionsmischung in Kontakt gebracht wird,
- wobei der rekombinante Mikroorganismus erhältlich ist, indem ein Wirtsorganismus, der Ketoisophoron zu Levodion reduzieren kann, ausgewählt aus der Gruppe bestehend aus Mikroorganismen der Gattungen *Saccharomyces, Zygosaccharomyces* und *Candida,* z.B. im Handel erhältliche Bäckerhefe, *Saccharomyces cerevisiae* ATCC7754, *Saccharomyces rouxii* HUT7191, *Zygosaccharomyces rouxii* IFO0494, *Saccharomyces delbrueckii* HUT7116, *Saccharomyces unisporus* IFO0298, *Saccharomyces delbrueckii (Torulaspora delbrueckii)* HUT7102, *Saccharomyces willianus* HUT7106, *Zygosaccharomyces bailii* ATCC11486, *Candida tropicalis* IFO1403 und eine Mutante davon, die Ketoisophoron zu Levodion reduzieren kann, mit einem Levodionreduktasegen transformiert wird, das aus einem Mikroorganismus stammt, der zu einer der Gattungen *Cellulomonas, Corynebacterium, Planococcus* und *Arthrobacter* gehört, z.B. *Cellulomonas* sp. AKU672 FERM BP-6449, *Corynebacterium aquaticum* AKU610 FERM BP-6447, *C. aquaticum* AKU611 FERM BP-6448, *P. okeanokoites* AKU152 IF015880, *A. sulfureus* AKU635 IFO12678 oder eine Mutante davon,
- wobei die Reaktion bei pH-Werten von 4,0 bis 9,0 und bei einer Temperatur im Bereich von 10 bis 50°C 15 Minuten bis 72 Stunden lang durchgeführt wird, und
das erzeugte Actinol aus der Reaktionsmischung isoliert wird.

2. Verfahren nach Anspruch 1, wobei die Reaktion bei pH-Werten von 5,0 bis 8,0 und bei einer Temperatur im Bereich von 20 bis 40°C 30 Minuten bis 48 Stunden lang durchgeführt wird.

3. Rekombinanter Mikroorganismus, der erhältlich ist, indem ein Wirtsmikroorganismus, der Ketoisophoron zu Levodion reduzieren kann, der zu den Gattungen *Saccharomyces, Zygosaccharomyces* oder *Candida* gehört, wie z.B. im Handel erhältliche Bäckerhefe, *S. cerevisiae* ATCC7754, *S*. *rouxii* HUT7191, *Z. rouxii* IFO0494, *S. delbrueckii* HUT7116, *S. unisporus* IFO0298, *S. delbrueckii (Torulaspora delbrueckii)* HUT7102, *S. willianus* HUT7106, *Z. bailii* ATCC11486, *C. tropicalis* IFO1403 und eine Mutante davon, die Ketoisophoron zu Levodion reduzieren kann, mit einem Levodionreduktasegen transformiert wird, das aus einem Mikroorganismus stammt, der zu einer der Gattungen *Cellulomonas, Corynebacterium, Planococcus* und *Arthrobacter* gehört, z.B. *Cellulomonas* sp. AKU672 FERM BP-6449, *Corynebacterium aquaticum* AKU610 FERM BP-6447, *C*. *aquaticum* AKU611 FERM BP-6448, *P. okeanokoites* AKU152 IFO15880, *A. sulfureus* AKU635 IFO12678 oder eine Mutante davon.

## Revendications

1. Procédé de production d'actinol à partir de cétoisophorone, qui comprend la mise en contact une cétoisophorone avec un microorganisme recombinant ou un extrait exempt de cellules de celui-ci dans un mélange de réaction,
- dans laquelle ledit microorganisme recombinant peut être obtenu en transformant un microorganisme hôte capable de réduire une cétoisophorone en lévodione, choisi à partir du groupe constitué des microorganismes des genres *Saccharomyces, Zygosaccharomyces,* et *Candida,* tels que la levure de boulanger disponible dans le commerce, *Saccharomyces cerevisiae* ATCC7754, *Saccharomyces rouxii* HUT7191, *Zygosaccharomyces rouxii* IFO0494, *Saccharomyces delbrueckii* HUT7116, *Saccharomyces unisporus* IFO0298, *Saccharomyces delbrueckii (Torulaspora delbrueckii)* HUT7102, *Saccharomyces willianus* HUT7106, *Zygosaccharomyces bailii* ATCC11486, *Candida tropicalis* IFO1403, et un mutant de ceux-ci qui est capable de réduire une cétoisophorone en lévodione, avec un gène de lévodione réductase, dérivé d'un microorganisme appartenant à l'un des genres *Cellulomonas, Corynebacterium, Planococcus, et Arthrobacter,* tel que *Cellulomonas* sp. AKU672 FERM BP-6449, *Corynebacterium aquaticum* AKU610 FERM BP-6447, *C.aquaticum* AKU61 FERM BP-6448, *P.okeanokoites* AKU152 IFO15880, *A.sulfureus* AKU635 IFO12678, ou un mutant de ceux-ci;
- dans laquelle la réaction est réalisée à des valeurs de pH de 4.0 à 9.0 et dans une gamme de température de 10 à 50°C et pendant 15 minutes à 72 heures, et l'isolement l'actinol produit à partir du mélange de réaction.

2. Procédé selon la revendication 1, dans lequel la réaction est réalisée à des valeurs de pH de 5.0 à 8.0, et dans une gamme de température de 20 à 40°C, et pendant 30 minutes à 48 heures.

3. Microorganisme recombinant pouvant être obtenu en transformant un microorganisme hôte capable de réduire une cétoisophorone en lévodione, appartenant aux genres *Saccharomyces, Zygosaccharomyces* ou *Candida,* tels que la levure de boulanger disponible dans le commerce, *S. cerevisiae* ATCC7754, *S. rouxii* HUT7191, *Z. rouxii* IFO0494, *S. delbrueckii* HUT7116, *S*. *unisporus* IFO0298, *S. delbrueckii (Torulaspora delbrueckii)* HUT7102, *S. willianus* HUT7106, *Z. bailii* ATCC11486, *C. tropicalis* IFO1403, et un mutant de ceux-ci qui est capable de réduire une cétoisophorone en lévodione, avec un gène de lévodione réductase, dérivé d'un microorganisme appartenant à l'un des genres *Cellulomonas, Corynebacterium, Planococcus, et* Arthrobacter, tel que *Cellulomonas* sp. AKU672 FERM BP-6449, *Corynebacterium aquaticum* AKU610 FERM BP-6447, *C. aquaticum* AKU611 FERM BP-6448, *P.okeanokoites* AKU152 IFO15880, *A.sulfureus* AKU635 IFO12678, ou un mutant de ceux-ci.


### SEQUENCE LISTING

<110> Roche Vitamins AG
   <120> Process for actinol production from ketoisophorone
   <130> NDR5223
   <160> 2
   <170> PatentIn version 3.1
<210> 1
   <211> 30
   <212> DNA
   <213> Artificial
<400> 1
   ggaggcgaat tcatgaccgc aaccagctcc
<210> 2
   <211> 27
   <212> DNA
   <213> Artificial
<400> 2
   gggctgctgc agtcagtacg cggcgga